# EUROPEAN PATENT APPLICATION

(11) **EP 4 035 677 A1**
(43) Date of publication of application: **03.08.2022**
(21) Application number: 21154077.8
(22) Date of filing: 28.01.2021
(51) Int. Cl.: A61K 39/215, C07K 14/165, C07K 16/10, C12N 7/02

(54) **FOLD PROMOTERS AND THEIR USE FOR THE PRODUCTION AND STABILIZATION OF POLYPEPTIDES**

(71) Applicant: Max-Planck-Gesellschaft zur Förderung der Wissenschaften e.V., 80539 München (DE)
(72) Inventor: The designation of the inventor has not yet been filed
(74) Representative: Weiss, Wolfgang

(57) **Abstract**

The present invention relates to the recombinant production of a protein of interest in a prokaryotic host cell wherein the protein of interest is obtained in a correctly folded and stable form. The protein of interest may be a difficult-to-make polypeptide for use as a vaccine or a pharmaceutical. The protein of interest is co-expressed with or fused to a 'fold promoter', which may be a VHH antibody recognizing the said protein.

## Description

The present invention relates to the recombinant production of a protein of interest in a prokaryotic host cell wherein the protein of interest is obtained in a correctly folded and stable form. The protein of interest may be a difficult-to-make polypeptide for use as a vaccine or a pharmaceutical. The protein of interest is co-expressed with or fused to a 'fold promoter', which may be a VHH antibody recognizing the said protein.

### Background

Newly synthesized proteins typically need to fold to become functional. This folding process involves forming (hydrogen-bonded) secondary structures (such as α-helices and β-sheets) and the burial of hydrophobic residues into the protein's hydrophobic core to acquire their tertiary structure (for review see: (Balchin et al., 2020; Trombetta and Parodi, 2003). Many secretory proteins, such as immunoglobulins, serum albumin, or RNase A, also form disulfide bonds from initially reduced cysteines. A failure to fold correctly leads to non-functional proteins, aggregation, and/or rapid degradation by dedicated proteases. Many proteins assemble into multi-subunit complexes (quaternary structure), examples being immunoglobulins or hemoglobin. Subunits, which cannot find an appropriate partner subunit, are typically eliminated by cellular quality control systems.

Protein folding is typically catalyzed by dedicated folding enzymes called chaperones, whereby the available chaperone repertoire depends on where folding occurs. The eukaryotic cytosol harbors numerous chaperones, including members of the Hsp70 and Hsp90 ATPases and the TRIC complex, which confines folding intermediates into a large folding chamber and releases them when folding is complete. Folding can occur already during synthesis (co-translational folding) or in a posttranslational manner.

Secretory proteins are transported in an unfolded state through a so-called translocon into the lumen of the endoplasmic reticulum (ER). These include numerous proteins used in pharmaceutical applications, such as immunoglobulins, immune-regulatory factors, or hormones. The folding environment of the ER is rather different from the cytosolic one: while the cytosol is reducing, the ER lumen allows stabilizing disulfide bonds to form - catalyzed by a set of specific enzymes that includes protein disulfide isomerases (PDIs). Likewise, folding inside the ER is often coupled to another ER-specific posttranslational modification, namely N-glycosylation, which enables glycosylation-assisted folding mechanisms involving lectin-like molecules. The ER is also capable of ATP-dependent protein folding, using ER-specific Hsp70 and Hsp90-type family members. However, the ER lacks GroEL/ Hsp60/ TRIC complex-type chaperones typical of the cytosolic folding environment. After having passed quality control, secretory proteins exit the ER, traverse the Golgi, where additional modifications such as proteolytic processing or changes to the sugar chains might occur.

Protein folding in prokaryotes, such as *Escherichia coli,* shows similarities but also differences to eukaryotic folding. Instead of the TRIC complex, the *E. coli* cytosol contains a smaller and simpler GroE-GroL complex. Secretion in *E. coli* typically ends in the periplasmic space, which harbors disulfide-bonding enzymes, but lacks glycosylation, glycosylation-coupled folding, and ATP-dependent folding enzymes.

Recombinant expression of proteins is not only important for basic research, but also it is a key process in the area of biotechnology, in particular for the production of pharmaceutically relevant biologicals. Several expression systems are available for recombinant expression. Cytoplasmic expression in *E.coli* is the easiest to implement - because of the fast growth and straightforward genetic manipulation of this bacterium, because of economic growth media, and its safe handling. However, many target proteins do not fold correctly in the *E.coli* cytosol, in particular targets that usually fold in a secretory compartment and rely on correct disulfide-bonding and/or glycosylation-assisted folding.

There are several solutions to this problem, such as switching to another expression system, e.g., to secretory expression from mammalian CHO cells. However, the latter is far more time consuming and expensive, particularly when it comes to large-scale expressions. Another solution is to manipulate the cytosolic folding system of bacteria such as *E.coli,* for example, by making this folding environment less reducing and by ectopic cytoplasmic expression of a periplasmic disulfide isomerase (DsbC) (Bessette et al., 1999; Levy et al., 2001). Meanwhile, such strains are commercially available, e.g., the *E.coli* NEB Express Shuffle strain from New England Biolabs.

However, even when using bacterial strains that have a modified cytosolic folding system, certain polypeptides are still not correctly folded. Thus there is a need for further improving the folding of recombinant proteins produced in a bacterial host cell.

### Summary of the Invention

A first aspect of the invention relates to a method for the recombinant production of a polypeptide of interest in a host cell, e.g. a prokaryotic host cell, or a eukaryotic host cell comprising the steps:
(a) providing a host cell comprising (i) a nucleic acid molecule encoding a fusion polypeptide comprising the polypeptide of interest and a fold promoter, or (ii) a first nucleic acid molecule encoding a polypeptide of interest and a second nucleic acid molecule encoding a fold promoter for the polypeptide of interest, wherein the fold promoter is a VHH antibody directed against the polypeptide of interest;
(b) cultivating the host cell under conditions where (i) the fusion polypeptide comprising the polypeptide of interest and the fold promoter is expressed or (ii) the polypeptide of interest and the fold promoter are co-expressed, and
(c) obtaining the polypeptide of interest in correctly folded form from the host cell.

In a particular embodiment, the polypeptide of interest is selected from a receptor binding domain (RBD) of a coronavirus spike protein, e.g. the RBD of the OC43, HKU1, 229E, NL63, SARS-CoV-1, MERS-CoV or SARS-CoV-2 spike protein.

A further aspect of the invention relates to a nucleic molecule encoding a fusion polypeptide comprising a polypeptide of interest and a fold promoter for the polypeptide of interest, wherein the fold promoter is a VVH antibody directed against the polypeptide of interest.

A further aspect of the invention relates to a set of nucleic molecules comprising a first nucleic acid molecule encoding a polypeptide of interest and a second nucleic acid molecule encoding a fold promoter for the polypeptide of interest, wherein the fold promoter is a VHH antibody directed against the polypeptide of interest.

A further aspect of the invention relates to a host cell comprising a nucleic molecule or a set of nucleic acid molecules as described above.

In a particular embodiment, the host cell is a prokaryotic host cell, e.g. an *E.coli* cell.

A further aspect of the invention relates to a polypeptide selected from a receptor binding domain (RBD) of a coronavirus spike protein, e.g. the RBD of the OC43, HKU1, 229E, NL63, SARS-CoV-1, MERS-CoV or SARS-CoV-2 spike protein, in correctly folded form, particularly produced in a prokaryotic host cell.

In a particular embodiment, the polypeptide is the RBD of the SARS-CoV-2 spike protein in correctly folded form, particularly produced in a prokaryotic host cell.

A further aspect of the invention relates to a complex of an RBD of a coronavirus spike protein and a fold-enhancing VHH antibody.

A further aspect of the invention relates to an RBD as described above for use in medicine.

A further aspect of the invention relates to a polypeptide of interest for use as a vaccine, wherein the polypeptide of interest is attached to a heterologous amino acid sequence having a length of at least about 5 amino acids, which has a negative net charge of at least about -5.

### Detailed Description

The present inventors explored the use of *E.coli* as a host cell for an economic recombinant production of the RBD (receptor-binding domain) of the SARS-CoV-2 spike protein. The RBD binds ACE2 during viral entry into target cells (Lan et al., 2020; Wang et al., 2020; Yan et al., 2020; Zhou et al., 2020a). Therefore, the RBD is a prime target for therapeutic intervention - be it for the production of neutralizing antibodies or as a constituent of vaccines. The RBD gets natively folded inside the ER lumen; it contains four structural disulfide bonds and gets N-glycosylated at two sites.

The inventors observed that the RBD failed to fold when expressed in the *E. coli* cytoplasm, even when using a strain that allows disulfide-bonding in the cytoplasm (NEB Shuffle). The small soluble fraction was then largely trapped in a non-productive complex with the chaperone GroE. This is in line with previous reports of failed folding of coronavirus RBDs in *E. coli* (Jegouic et al., 2020; Chen et al., 2005). Failure of correct folding was further confirmed by the finding that the soluble RBD fraction was not recognized by the super-neutralizing anti-RBD VHH antibody Re5D06, which is disclosed among a large set of other anti-RBD VHH antibodies in EP 20 188 541, EP 21 151 145, and EP 21 151 159, the contents of which are herein incorporated by reference.

Additional attempts of testing numerous variants of periplasmic RBD expression also gave unacceptably low yields. The still best variant was a fusion to the periplasmic disulfide interexchange protein G (DsbG), which yielded a soluble DsbG-RBD fusion that behaved in gel filtration like a folded protein. Nevertheless, the periplasmic expression workflow requires an osmotic shock protocol to release the periplasmic fraction, which cannot be easily implemented for an industrial-scale production; and yields were moderate at best.

The inventors discovered that a correct folding of the RBD domain could be enforced in the *E.coli* cytosol by its co-expression or fusion with "fold-promoting" anti-RBD VHH antibodies.

To this end, the inventors explored numerous ways to obtain a correctly folded RBD through expression in the *E.coli* cytosol. Eventually, the inventors tried the unconventional approach of testing systematically if co-expressing specific VHH antibodies could force the RBD to fold properly in the NEB Shuffle Express strain. Indeed, amongst 32 tested anti-RBD VHH antibodies, seven had the desired RBD-fold-promoting effect, namely: Re6A11, Re6B07, Re7E02, Re9C07, Re9D02, Re9F06, Re9G12 and Re11H04.

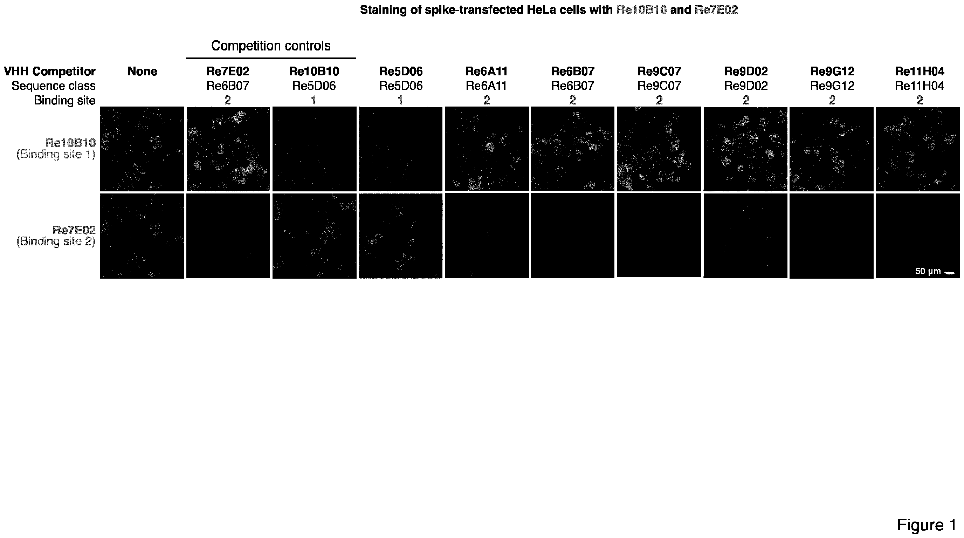

All of these seven VHH antibodies compete for the same RBD epitope (defined by Re7E02; see Figure 1). By binding their epitope, it appears that these VHH antibodies stabilize an early folding intermediate and thereby block the divergence to non-productive folding paths. This "fold-promoter" epitope is non-overlapping with the epitope of the super-neutralizing VHH Re5D06 (Figure 1), i.e., a fold-promoting VHH and Re5D06 can bind the same RBD molecule at the same time.

Accordingly, the present invention provides a covalent fusion polypeptide of a protein of interest, e.g. a coronavirus RBD and a fold promoting VHH antibody, a non-covalent complex of a protein of interest, e.g. a coronavirus RBD, and a fold promoting VHH antibody. The covalent fusion polypeptide optionally comprises further domains, e.g. a purification domain, e.g. an N-terminal cleavable purification tag, and/or a spacer located between the polypeptide of interest and the fold promoting VHH antibody.

The term "protein of interest" generally relates to any globular protein, protein fragment or protein domain that folds inefficiently when produced in a heterologous system.

The term "fold-enhancing VHH antibody" refers to a VHH antibody, which when co-expressed or fused to the protein of interest leads to an increase of the amount of correctly folded protein when expressed in a host cell, particularly in a prokaryotic host cell such as *E. coli.*

A "non-competing antibody" may be VHH antibody, or any other type of antibody or antigen-binding fragment thereof, and is directed against an epitope on the protein of interest, which is different from the epitope, against which the fold-enhancing VHH antibody is directed. Preferably, the non-competing antibody is a monovalent antibody, i.e. an antibody having a single antigen-binding site. More preferably, the non-competing antibody is a VHH antibody. The non-competing antibody and the fold-enhancing VHH antibody can bind to the protein of interest simultaneously. In certain embodiments, the non-competing antibody selectively binds to the protein of interest in correctly folded form, e.g. by binding to a conformational epitope on the protein of interest

A list of fold-enhancing VHH antibodies Re6A11, Re6B07, Re7E02, Re9C07, Re9D02, Re9F06, Re9G12, and Re11H04, and of the non-competing VHH antibody R6D05 and their CDR1, CDR2, and CDR3 sequences is shown in the following Table 1:

**Table 1**

| VHH | SEQ ID NO. | CDR1 | SEQ ID NO. | CDR2 | SEQ ID NO. | CDR3 | SEQ ID NO. |
|---|---|---|---|---|---|---|---|
| Re6A11 | 2 | RTFSND ALG | 3 | FVAAINWNSGTYY A | 4 | AAASDYGLPREDFLYDYW | 5 |
| Re6B07 | 6 | FTLDYYA IG | 7 | GVSYIRSSDGTTY YA | 8 | AADEAYYSELGWESPWGW SYW | 9 |
| Re7E02 | 10 | FTLDYYA IG | 11 | GVSYIRSSDGTTY YA | 12 | AADEAYYSELGWESPWGW SYW | 13 |
| Re9C07 | 14 | RTFSRY AMG | 15 | FVAAITWNADTTY YA | 16 | AAGGNHYYSRSYYSSLEYD HW | 17 |
| Re9D02 | 18 | RTFSNY AMG | 19 | FVAAISWGGDTTY YA | 20 | AADRGLSYYYDRVTEYDY W | 21 |
| Re9F06 | 22 | RTFSND ALG | 23 | FVAAINWNSGTYY A | 24 | AAASDYGLPREDFLYDYW | 25 |
| Re9G12 | 26 | RTFSSY VMG | 27 | FVAHISWSGDSTY YA | 28 | AADRGASYYYTWASEYNY W | 29 |
| Re11H04 | 30 | FTLDYYT IA | 31 | GVSCISGNDGSTY YA | 32 | AADRGESYYPFRPSEYHY W | 33 |
| | | | | | | | |
| Re5D06 | 34 | ITLDYYAI G | 35 | GVSRIRSSDGSTN YA | 36 | AYGPLTKYGSSWYWPYEY DYW | 37 |

The VHH antibody as described herein is particularly a monoclonal VHH antibody characterized by a specific amino acid sequence. The VHH antibody may be produced in a prokaryotic host cell, a yeast cell or a mammalian cell. In certain embodiments, the VHH antibody is non-glycosylated. In certain embodiments, the VHH antibody is glycosylated, wherein a carbohydrate structure may be derived from a glycosylation site introduced into the VHH sequence and/or from a fusion partner.

A VHH antibody is characterized by (i) a CDR3 sequence, (ii) a combination of a CDR1 sequence, a CDR2 sequence, and a CDR3 sequence, (iii) by a complete VHH sequence, or (iv) by competition with a specific reference antibody. Specific CDR and VHH sequences are provided in the Tables, Figures and the Sequence Listing. According to the present invention, sequences related to the above sequences are encompassed. These related sequences are defined by having a minimum identity to a specifically indicated amino acid sequence, e.g. a CDR or VHH sequence. This identity is indicated over the whole length of the respective reference sequence and may be determined by using well known algorithms such as BLAST.

In particular embodiments, a related CDR3 sequence has an identity of at least 80% or at least 90% or at least 95% to a specifically indicated CDR3 sequence, e.g. a substitution of 1, 2, or 3 amino acids.

In particular embodiments, a related combination of a CDR1 sequence, a CDR2 sequence, and a CDR3 sequence has an identity of at least 80% or at least 90% or at least 95% to a specifically indicated combination of a CDR1 sequence, a CDR2 sequence, and a CDR3 sequence, e.g. a substitution of 1, 2, 3, 4, 5 or 6 amino acids by different amino acids.

In particular embodiments, a related VHH sequence has an identity of least 70%, at least 80%, at least 90%, at least 95% or at least 99% to a VHH sequence, e.g. a substitution of 1, 2, 3, 4, 5 or up to 20 amino acids.

Further, the invention refers to a VHH antibody, which competes with a specific VHH antibody disclosed herein for the binding to the SARS-CoV-2 spike protein S1 domain. In certain embodiments, a competing VHH antibody binds the same or an overlapping epitope on the SARS-CoV-2 spike protein S1 domain, particularly the receptor-binding domain (RBD) of the SARS-CoV-2 S1 domain as a specific VHH antibody disclosed herein. For example, the invention refers to a fold-enhancing VHH antibody, which competes with VHH antibodies Re6B07 or Re7E02.

Competition is measured either as an at least 90% loss of fluorescence staining of the polypeptide of interest, e.g. the SARS-CoV-2 spike protein by a fluorophore-labelled candidate VHH antibody in the presence of a 100-fold molar excess of an unlabelled competitor VHH antibody, e.g. Re7E02, or conversely as an at least 90% loss of polypeptide staining, e.g. spike protein-staining by a fluorophore-labelled VHH antibody, e.g. Re7E02 in the presence of a 100-fold molar excess of the unlabelled candidate VHH antibody as a competitor.

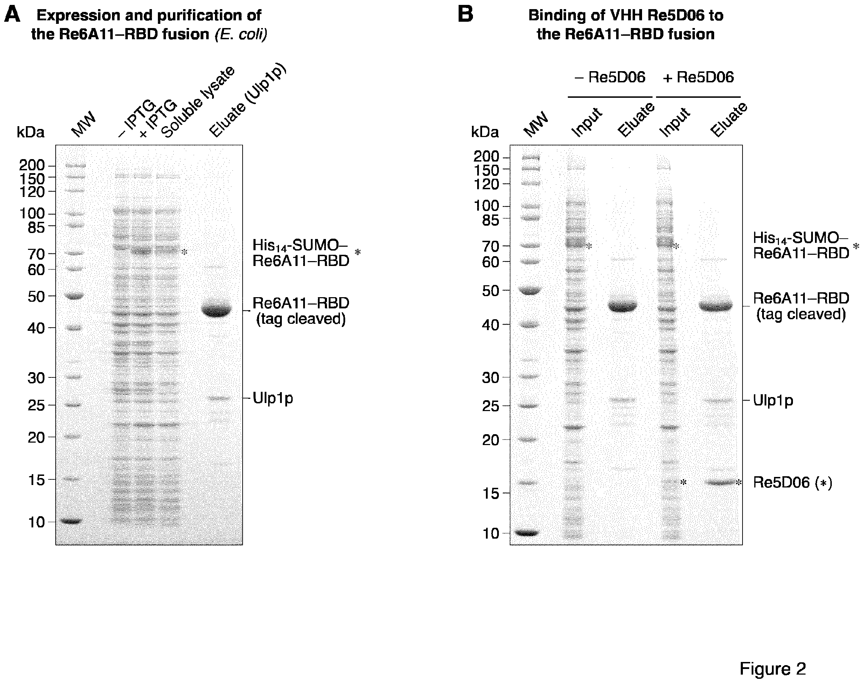

In their first experiments, the inventors co-expressed the VHH antibody and the RBD from two different plasmids. This was not quite ideal as the expression levels of the two polypeptides turned out to be different. Therefore, the presently preferred solution is to express the fold promoter and the RBD as a single fusion polypeptide comprising (i) optionally a cleavable N-terminal purification tag, e.g. the His14-SUMO module, (ii) the fold-promoting VHH, (iii) a spacer, e.g. a 39 residues long flexible, hydrophilic and negatively charged spacer, and (iv) the RBD. Such an entity (a His14-SUMO-VHH Re6A11-spacer-SARS-CoV-2-RBD fusion) was found to be well expressed in the cytoplasm of *E. coli* NEB Shuffle, soluble and purifiable in a single Ni (II) chelate step (Figure 2A).

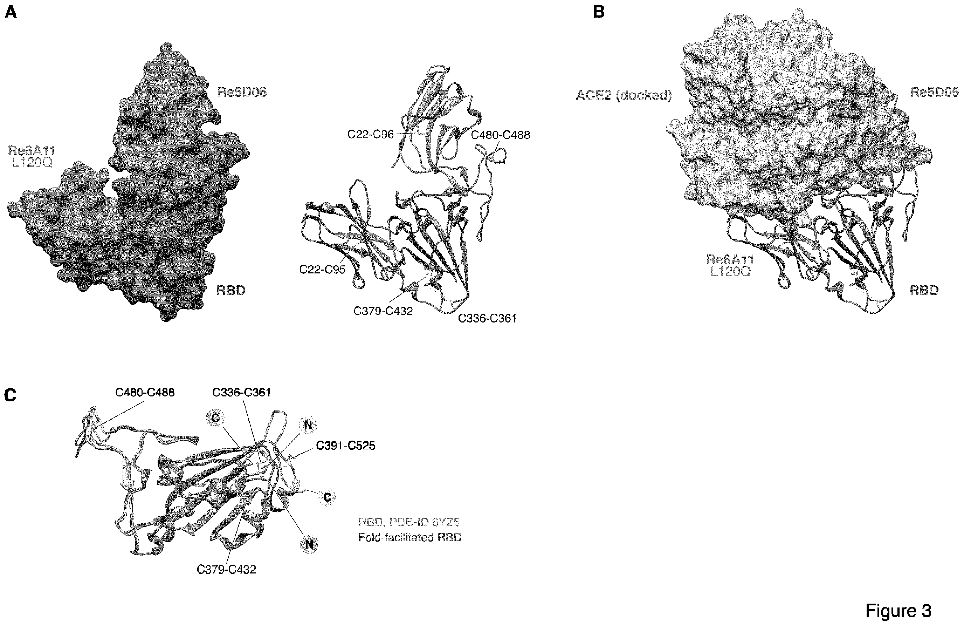

The interpretation of proper RBD-folding in the presence of those VHH antibodies rests on several lines of evidence. First, their co-expression or fusion increased soluble RBD expression. Second, it counteracted trapping by GroE. Third, binary fold-promoting VHH-RBD complexes or VHH-RBD fusions were easily obtained by co-expression and behaved, e.g., during gel filtration, like properly folded entities. Fourth, those binary complexes bound a non-competing antibody, e.g. complementary VHH Re5D06 with high affinity and specificity (Figure 2B). As Re5D06 or an antibody competing therewith, e.g. as disclosed in EP 20 188 541, EP 21 151 145, and EP 21 151 159, recognizes a conformational epitope (see below Figure 3), this again is evidence for proper folding.

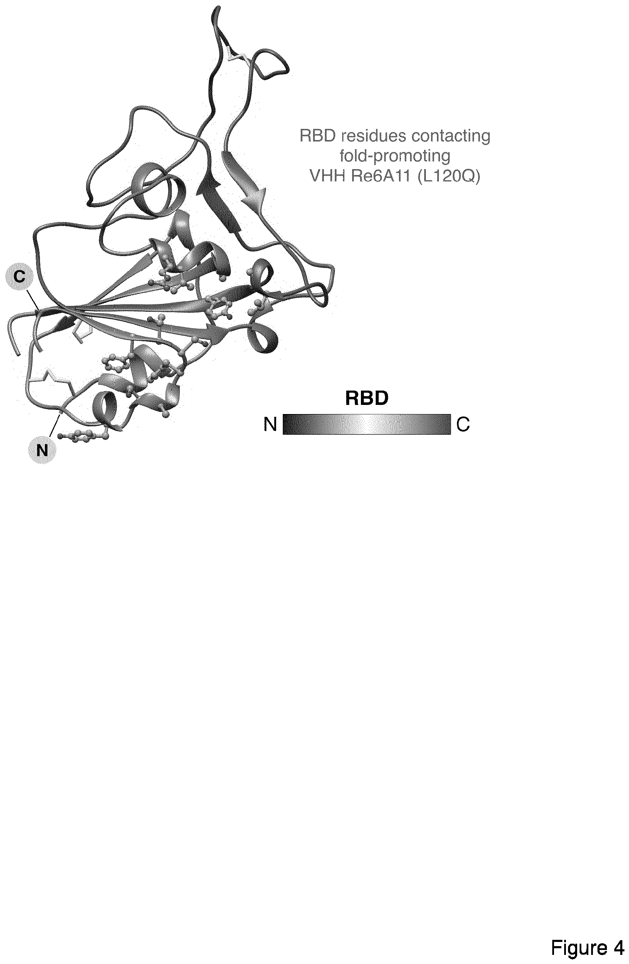

To obtain ultimate proof for correct folding of the RBD obtained under such expression conditions, the inventors crystallized a trimeric complex comprising the fold-promoting VHH antibody Re6A11, the RBD, and the neutralizing VHH antibody Re5D06 and solved the structure of this complex to a resolution of 1.75Å (Figure 3A-C). The structure indeed showed the correct fold of the RBD, including appropriate disulfide bonds. Figure 3C visualizes this by overlaying the RBD from the fold-facilitator complex with a previously solved RBD structure (PDB 6YZ5). Figure 4 shows the residues of the RBD, which are contacted by the fold-facilitator Re6A11. It appears as if the stabilization of this conformational epitope by VHH Re6A11 allows the RBD to complete the folding process properly. One can also rationalize the fold-promoting effect by considering that the VHH binding prevents the folding intermediate from getting trapped in a non-productive GroE complex. From yet a different perspective, one can see it as an artificial, stabilizing subunit of the RBD.

Another important detail is that the fold promoter produces only a comparably small clash with ACE2, which is sufficient to preclude ACE2-binding but leaves most of the ACE2-binding interface of the RBD exposed (Figure 3B). In other words, the fold-promoter supports folding of the RBD without obstructing the interface that should be neutralized by vaccine-triggered antibodies.

The fold promoter Re6A11 not only leaves the best epitope for neutralization fully exposed but should even improve its presentation to the immune system because it prevents masking of this epitope by ACE2 (Figure 3B). In fact, this might even reduce side effects of the immunization caused by the undesired binding of an RBD-vaccine to ACE2-presenting cells with subsequent antibody-binding/ opsonization of those cells.

In the aforementioned Re6A11-RBD fusion, the inventors linked the two entities with a long, negatively charged spacer of 39 residues in length and a net charge of -13. The length is needed to safely bridge the 56 Å distance between the C-terminus of Re6A11 and the N-terminus of the RBD. The negatively charged spacer serves, however, potentially yet another function, namely, to confer binding to cationic lipids or Al³⁺-based colloids that are typical components/carriers of vaccine formulations. This binding should concentrate the antigen on the surface of the particulate carriers and thus increase immunogenicity by presenting the antigen in a multivalent form.

The present invention provides a fusion polypeptide comprising a polypeptide of interest and a VHH antibody further comprising a heterologous amino acid sequence as a spacer having a length of at least about 5 amino acids and preferably up 100 amino acids, particularly about 5 to 50 amino acids. In certain embodiments, this polypeptide is selected from viral antigens that might trigger a neutralizing antibody response in humans or animals. The spacer has a negative net charge, particularly a negative net charge of at least about -5, more particularly a negative net charge of about -8 to about -30 or even more. The negative net charge of the spacer results from an excess of negatively charged amino acids therein. Preferably, the spacer has a content of at least about 15%, at least about 20% or least about 25% of negatively charged amino acids such as Asp and Glu. Further, the spacer may have a content of at least about 80%, or at least about 90% and up to about 100 % of hydrophilic amino acids, particularly selected from Gly, Ser, Glu, Asp, Thr, Pro, and Gln.

The RBD co-expression with a fold-stabilizing VHH antibody as fold promoter or the RBD expression as a fusion with a fold-stabilizing VHH antibody a host cell like E. *coli* is thus a viable option to produce an anti-SARS-CoV-2 vaccine.

One can expand this concept to other vaccine candidates, such as RBDs from other coronaviruses that cause infections in humans (OC43, HKU1, 229E, NL63, SARS1, and MERS) or farm animals (e.g., swine and bovine coronaviruses).

The concept can be further expanded to other biotechnologically relevant biologicals, where a VHH can assist folding and production without interfering with the intended downstream application.

Further, the present invention provides a polypeptide of interest, particularly for use as a vaccine, wherein the polypeptide is attached to a heterologous amino acid sequence having a length of at least about 5 amino acids and preferably up 100 amino acids, particularly about 5 to 50 amino acids. In certain embodiments, this polypeptide is selected from viral antigens that might trigger a neutralizing antibody response in humans or animals. The heterologous amino acid sequence has a negative net charge, particularly a negative net charge of at least about -5, more particularly a negative net charge of about -8 to about -30 or even more. The negative net charge of the heterologous amino acid sequence results from an excess of negatively charged amino acids therein. Preferably, the heterologous amino acid sequence has a content of at least about 15%, at least about 20% or least about 25% of negatively charged amino acids such as Asp and Glu. Further, the heterologous amino acid sequence may have a content of at least about 80%, or at least about 90% and up to about 100 % of hydrophilic amino acids, particularly selected from Gly, Ser, Glu, Asp, Thr, Pro, and Gln.

As indicated above, the heterologous spacer and the heterologous amino acid sequence have a negative net charge. This will increase the interaction with a positively charged compound, such as a polyamine, e.g. spermidine, a cationic polypeptide, e.g. protamine, a cationic lipid or an Al³⁺-based colloid or a particle comprising such a positively charged compound. Such a positively charged compound or particle is a typical component and/or carrier of a vaccine formulation. By means of this interaction, a non-covalent complex of the polypeptide and a positively charged compound or a particle comprising a positively charged compound will be generated. Thereby, the polypeptide will be concentrated on the surface of a positively charged particulate carrier and thus increase immunogenicity by presenting the antigen in a multivalent form.

Thus, the invention provides a non-covalent complex of a polypeptide comprising a heterologous spacer or a heterologous amino acid sequence as described and positively charged compound including a particle comprising a positively charged compound. This complex is particularly useful as a component of immunogenic composition, e.g. an immunogenic composition for use as vaccine, e.g. in human medicine or in veterinary medicine, or for use in the production of antibodies, e.g. in an experimental animal.

In the following, particular embodiments of the specification are listed.

### Embodiments of the Specification

1. A method for the recombinant production of a polypeptide of interest in a host cell, e.g. a prokaryotic host cell, or a eukaryotic host cell comprising the steps:
   (a) providing a host cell comprising (i) a nucleic acid molecule encoding a fusion polypeptide comprising the polypeptide of interest and a fold promoter, or (ii) a first nucleic acid molecule encoding a polypeptide of interest and a second nucleic acid molecule encoding a fold promoter for the polypeptide of interest, wherein the fold promoter is selected from:
      (i) a VHH antibody directed against the polypeptide of interest; and
      (ii) a periplasmic disulfide interexchange protein G (DsbG);
   (b) cultivating the host cell under conditions where (i) the fusion polypeptide comprising the polypeptide of interest and the fold promoter is expressed or (ii) the polypeptide of interest and the fold promoter are co-expressed, and
   (c) obtaining the polypeptide of interest in correctly folded form from the host cell.
2. The method of item 1, wherein the host cell is a prokaryotic host cell, e.g. a Gram-negative prokaryotic cell such as *E. coli* or a Gram-positive prokaryotic cell such as *Bacillus ssp.*
3. The method of item 1, wherein the host cell is a eukaryotic host cell, e.g. a yeast cell, an insect cell or a mammalian cell.
4. The method of any one of items 1-3, wherein polypeptide of interest is expressed as a fusion polypeptide.
5. The method of any one of items 1-4, wherein the nucleic acid molecule (i) encodes a fusion polypeptide further comprising an N-terminal purification tag, particularly a cleavable tag, e.g. a poly-His tag, a cleavable poly-His-SUMO tag or a cleavable poly-His-NEDD8 tag.
6. The method of any one of items 1-5, wherein the nucleic acid molecule (i) encodes a fusion polypeptide further comprising a heterologous spacer between the polypeptide of interest and the fold promoter.
7. The method of item 6, wherein the spacer has a length of at least about 5 amino acids and up to about 100 amino acids, particularly about 5 to about 50 amino acids.
8. The method of item 7, wherein the spacer has a negative net charge of at least -5, and particularly a content of at least about 15%, at least about 20% or least about 25% of negatively charged amino acids selected from Asp and Glu.
9. The method of any one of items 6-8, wherein the spacer has a content of at least about 80%, or at least about 90% and up to about 100 % of hydrophilic amino acids, particularly selected from Gly, Ser, Glu, Asp, Thr, Pro, and Gln.
10. The method of any one of items 1-9, wherein the polypeptide of interest is expressed as a fusion polypeptide and wherein the polypeptide of interest is cleaved from the fold promoter or wherein the polypeptide is obtained as a fusion polypeptide.
11. The method of any one of items 1-3, wherein the polypeptide of interest and the fold promoter are expressed as separate polypeptides.
12. The method of any one of the preceding items, wherein the polypeptide of interest is the RBD of a coronavirus spike protein, e.g. the RBD of the SARS-Cov-1 spike protein, the RBD of the MERS-CoV spike protein, or the RBD of the SARS-CoV-2 spike protein.
13. The method of item 12, wherein the polypeptide of interest is the RBD of the SARS-CoV-2 spike protein, particularly having an amino acid sequence as shown in SEQ: ID NO. 1 or a sequence identity of at least 80%, at least 90% or at least 95% thereto.
14. The method of item 12 or 13, wherein the fold promoter is selected from a VHH antibody comprising
   (a) a CDR3 sequence as shown in SEQ ID NO. 5, 9, 13, 17, 21, 25, 29, or 33,
   (b) a CDR3 sequence, which has an identity of at least 80%, at least 90% or at least 95% to a CDR3 sequence of (a), or
   (c) a VHH antibody, which competes with a VHH antibody of (a) for the binding to the SARS-CoV-2 spike protein S1 domain, particularly the receptor-binding domain (RBD) of the SARS-CoV-2 S1 domain.
15. The method of any one of items 12-14, wherein the fold promoter is selected from a VHH antibody comprising
   (a) a combination of CDR1, CDR2 and CDR3 sequences as shown in SEQ ID NO. 3-5, 7-9, 11-13, 15-17, 19-21, 23-25, 27-29, or 31-33,
   (b) a combination of CDR1, CDR2 and CDR3 sequences which has an identity of at least 80%, at least 90% or at least 95% to a combination of CDR1, CDR2 and CDR3 sequences of (a), or
   (c) a VHH antibody, which competes with a VHH antibody of (a) for the binding to the SARS-CoV-2 spike protein S1 domain, particularly the receptor-binding domain (RBD) of the SARS-CoV-2 S1 domain.
16. The method of any one of items 12-15, wherein the fold enhancer is selected from a VHH antibody comprising
   (a) a VHH sequence as shown in SEQ ID NO. 2, 6, 10, 14, 18, 22, 26, or 30,
   (b) a sequence which has an identity of at least 70%, at least 80%, at least 90%, at least 95% or at least 99% to a VHH sequence of (a), or
   (c) a VHH antibody, which competes with a VHH antibody of (a) for the binding to the SARS-CoV-2 spike protein S1 domain, particularly the receptor-binding domain (RBD) of the SARS-CoV-2 S1 domain.
17. The method of any one of items 1-10 and 12-16, wherein the fold promoter is selected from the periplasmic disulfide interexchange protein G (DsbG) as shown in SEQ: ID NO. 41 or a sequence identity of at least 80%, at least 90% or at least 95% thereto.
18. A nucleic molecule encoding a fusion polypeptide comprising a polypeptide of interest and a fold promoter for the polypeptide of interest, wherein the fold promoter is selected from:
   (i) a VVH antibody directed against the polypeptide of interest; and
   (ii) a periplasmic disulfide interexchange protein G (DsbG).
19. A vector comprising the nucleic acid molecule of item 18 in operative linkage with an expression control sequence.
20. A set of nucleic molecules comprising a first nucleic acid molecule encoding a polypeptide of interest and a second nucleic acid molecule encoding a fold promoter for the polypeptide of interest, wherein the fold promoter is selected from:
   (i) a VVH antibody directed against the polypeptide of interest; and
   (ii) a periplasmic disulfide interexchange protein G (DsbG).
21. A vector or a set of vectors comprising the first nucleic acid molecule and the second nucleic acid molecule of item 20 each in operative linkage with an expression control sequence.
22. A host cell comprising a nucleic acid molecule of item 18, a vector of item 19, a set of nucleic acid molecules of item 20 or a vector or a set of vectors of item 21.
23. The host cell of item 22, wherein the nucleic acid molecule encoding the polypeptide of interest is extrachromosomal.
24. The host cell of item 22, wherein the nucleic acid molecule encoding the polypeptide of interest is chromosomally integrated.
25. An RBD of a coronavirus spike protein, e.g. the RBD of the OC43, HKU1, 229E, NL63, SARS-CoV-1, MERS-CoV or SARS-CoV-2 spike protein, in correctly folded form, particularly produced in a prokaryotic host cell.
26. An RBD of the SARS-CoV-2 spike protein, particularly having an amino acid sequence as shown in SEQ: ID NO. 1 or a sequence identity of at least 80%, at least 90% or at least 95% thereto, in correctly folded form, particularly produced in a prokaryotic host cell.
27. The RBD of item 26, which binds to VHH antibody comprising
   (a) a CDR3 sequence as shown in SEQ ID NO. 37 or a CDR3 sequence, which has an identity of at least 80%, at least 90% or at least 95% thereto,
   (b) a combination of CDR1, CDR2 and CDR3 sequences as shown in SEQ ID NO. 35-37 or a combination of CDR1, CDR2 and CDR3 sequences, which has an identity of at least 80%, at least 90% or at least 95% thereto,
   (c) a VHH sequence as shown in SEQ ID NO. 34, or
   (d) a VHH antibody, which competes with a VHH antibody of (a), (b) or (c) for the binding to the SARS-CoV-2 spike protein S1 domain, particularly the receptor-binding domain (RBD) of the SARS-CoV-2 S1 domain.
28. A fusion protein of an RBD of a coronavirus spike protein, e.g. the RBD of the OC43, HKU1, 229E, NL63, SARS-CoV-1, MERS-CoV or SARS-CoV-2 spike protein, and a fold-enhancing VHH antibody.
29. The fusion protein of item 28 comprising the RBD of the SARS-CoV-2 spike protein, particularly having an amino acid sequence as shown in SEQ: ID NO. 1 or a sequence identity of at least 80%, at least 90% or at least 95% thereto, and a fold-enhancing VHH antibody.
30. A non-covalent complex of an RBD of a coronavirus spike protein, e.g. the RBD of the OC43, HKU1, 229E, NL63, SARS-CoV-1, MERS-CoV or SARS-CoV-2 spike protein, and a fold-enhancing VHH antibody.
31. The non-covalent complex of item 30 comprising the RBD of the SARS-CoV-2 spike protein, particularly having an amino acid sequence as shown in SEQ: ID NO. 1 or a sequence identity of at least 80%, at least 90% or at least 95% thereto, and a fold-enhancing VHH antibody.
32. A fusion protein comprising an RBD of a coronavirus spike protein, e.g. the RBD of the OC43, HKU1, 229E, NL63, SARS-CoV-1, MERS-CoV or SARS-CoV-2 spike protein and a periplasmic disulfide interexchange protein G (DsbG), in correctly folded form, particularly produced in a prokaryotic host cell.
33. The fusion protein of item 32 comprising an RBD of the SARS-CoV-2 spike protein, particularly having an amino acid sequence as shown in SEQ: ID NO. 1 or a sequence identity of at least 80%, at least 90% or at least 95% thereto, and a periplasmic disulfide interexchange protein G (DsbG), in correctly folded form, particularly produced in a prokaryotic host cell.
32. An RBD of any one of items 25-27 or 30-31 for use in medicine.
33. An RBD of any one of items 25-27 or 30-31 for use as a vaccine.
34. An RBD of any one of items 25-27 or 30-31 for use in the production of antibodies, particularly neutralizing antibodies.
35. A fusion polypeptide comprising a polypeptide of interest and a VHH antibody further comprising a heterologous amino acid sequence as a spacer having a length of at least about 5 amino acids and preferably up 100 amino acids, which has a negative net charge and particularly a content of at least about 15%, at least about 20% or least about 25% of negatively charged amino acids selected from Asp and Glu.
36. A polypeptide for use as a vaccine, which is attached to a heterologous amino acid sequence having a length of at least about 5 amino acids and particularly up to about 100 amino acids, which has a negative net charge of at least -5 and particularly a content of at least about 15%, at least about 20% or least about 25% of negatively charged amino acids selected from Asp and Glu.
37. A non-covalent complex of a polypeptide of item 35 or 36 and a positively charged compound or a particle comprising a positively charged compound.

### Examples and Figures

**Figure 1****: All fold-promoting VHHs compete for the same epitope on the RBD.** HeLa cells were transiently transfected to express the SARS-CoV-2 spike protein. Following fixation, cells were stained for 1 hour with fluorophore-labeled Re10B10 (5 nM, green) and Re7E02 (15 nM, red) in the presence of the indicated unlabeled VHH competitors. Competitor (150 nM) was added 20 min prior to the labeled nanobodies. Cells were imaged by confocal laser scanning microscopy. For each competitor, the binding site on the RBD (epitope 1 or 2) are indicated. Re10B10 and Re5D06 define the competition class for epitope 1, while the fold enhancers Re6A11, Re6B07, Re7E02, Re9C07, Re9D02, Re9G12, and Re11H04 compete for epitope 2 and thus bind to a different site.
**Figure 2****: Soluble expression and purification of the Re6A11-RBD fusion.**
   **A)** An expression plasmid was constructed that encodes a fusion with an N-terminal His-SUMO tag, VHH-Re6A11, a 39 residues long acidic spacer (net charge: -13), and the SARS-CoV-2 RBD. *E. coli* NEB Express Shuffle was transformed with this plasmid, and plasm id-containing transformants were selected with 50 µg/ml kanamycin. Bacteria were grown in TB medium, and T5/lac-controlled expression was induced at 25°C with 100 µM IPTG for 3 hours. Cells were recovered by centrifugation, resuspended in 50 mM Tris/HCI pH 7.5, 300 mM NaCl, 20 mM imidazole/ HCI pH 7.5, and lysed by ultrasonication. The soluble fraction was obtained by ultracentrifugation. 45 ml soluble fraction (corresponding to 750 ml culture) was bound to a 1.2 ml Ni(II) EDTA-amide chelate column. Non-bound material was washed off with sonication buffer, and the Re6A11-RBD fusion was eluted by cleaving the His-SUMO-tag with the SUMO Ulp1 protease. Panel shows analysis of the experiment by SDS-PAGE (Coomassie-staining). Note the strong Re6A11-RBD fusion band in the eluted fraction. The yield was approximately 8 mg Re6A11-RBD fusion per liter of culture.
   **B)** An analytical version of the experiment from panel a. Where indicated, 1 µM untagged Re5D06 was added to the binding reaction; elution was with lysis buffer containing 500 mM imidazole, which leaves the His₁₄-SUMO fusion intact. Co-elution of Re5D06 with His14-SUMO-Re6A11-RBD indicates a specific RBD·Re5D06 interaction. The endogenous *E. coli* proteins present in the soluble starting lysate can be considered as negative controls for the binding reaction.
**Figure 3****: High-resolution crystal structure of the ternary Re9F06·RBD·Re5D06 complex.**
   The complex was produced by co-expressing all three components in the cytoplasm of *E. coli* NEB Shuffle Express, then purified by double-tag purification (Frey and Gorlich, 2014a; Frey and Gorlich, 2014b) followed by gel filtration. The homogeneous complex was crystallized, an x-ray diffraction dataset was recorded at the Swiss Light Source synchrotron, and the structure solved by molecular replacement to a resolution of 1.75 Å and an Rfree of 0.229.
   **A)** Crystal structure of the Re9F06·PBD·Pe5D06 complex in surface (left) or ribbon representation (right). The intramolecular disulfide bonds (shown as yellow sticks) are labeled.
   **B)** Ribbon representation of the Re9F06·RBD·Re5D06 complex as in **A**, but with RBD-bound ACE2 shown as transparent brown surface. Docking of ACE2 is based on alignment of the RBD with the SARS-CoV-2 RBD·ACE2 complex (PDB ID 7KMS; (Zhou et al., 2020b); RMSD=0.986 Å). Note the frontal clash of ACE2 with Re5D06. The clash between Re9F06 and ACE2 is minor and caused by CDR2 and the VHH scaffold protruding into ACE2; despite the clash, Re9F06 and ACE2 contact different residues on the RBD.
   **C)** Comparison of the RBD structure in the ternary Re9F06·RBD·Re5D06 complex with the structure of the RBD as reported by PDB 6YZ5. Note that the folds are identical, with only minor deviations in some flexible loop structures.
**Figure 4****: The fold-promoter Re6A11 recognizes a conformational RBD epitope**
   The SARS-CoV-2 RBD from Figure 3 is shown as a ribbon colored according to the indicated color gradient, with its N-terminus in blue and C-terminus in red. Disulfide bridges are depicted as yellow sticks. Side chains that interact with the fold-promoting VHH Re9F06 are shown in green, as ball-and-sticks.

### Sequences

SEQ ID NO. 1: SARS-CoV-2 RBD
PNITNLCPFGEVFNATRFASVYAWNRKRISNCVADYSVLYNSASFSTFKCYGVSPT
KLNDLCFTNVYADSFVIRGDEVRQIAPGQTGKIADYNYKLPDDFTGCVIAWNSNNLD
SKVGGNYNYLYRLFRKSNLKPFERDISTEIYQAGSTPCNGVEGFNCYFPLQSYGFQ
PTNGVGYQPYRVVVLSFELLHAPATVCGP

SEQ ID NO. 2: Re6A11
GSQVQLVESGGGLVQAGGSLRLSCAASGRTFSNDALGWFRQAPRKEREFVAAIN
WNSGTYYADSVKGRFTISRDNAKNTVYLQMNSLKPEDTAVYSCAAASDYGLPRED
FLYDYWGQGTLVTVSSTS

SEQ ID NO. 6: Re6B07
GSQVQLVESGGGLVQPGGSLRLSCAASGFTLDYYAIGWFRQAPGKEREGVSYIRS
SDGTTYYADSVKGRFTISRDNAKNTVYLQMNSLKPEDTAVYYCAADEAYYSELGW
ESPWGWSYWGQGTRVTVSSTS

SEQ ID NO. 10: Re7E02
GSQVQLVESGGGLVQAGGSLRLSCAASGFTLDYYAIGWFRQAPGKEREGVSYIRS
SDGTTYYADSVKGRFTISRDNAKNTVYLQMNSLKPEDTAVYYCAADEAYYSELGW
ESPWGWSYWGQGTQVTVSSTS

SEQ ID NO. 14: Re9C07
GSQVQLVESGGGLVQAGGSLRLSCAASGRTFSRYAMGWFRQAPGKEREFVAAIT
WNADTTYYADSVKGRFTISRDNAKNTVYLQMNSLKPEDTAVYYCAAGGNHYYSRS
YYSSLEYDHWGQGTQVTVSSTS

SEQ ID NO. 18: Re9D02
GSQVQLVESGGGLVQAGGSLRLSCAASGRTFSNYAMGWFRQAPGKEREFVAAIS
WGGDTTYYADSLKGRFTISRDNAKNTVYLQMNSLKPEDTAVYYCAADRGLSYYYD
RVTEYDYWGQGTQVTVSSTS

SEQ ID NO. 22: Re9F06
GSQVQLVESGGGLVQAGGSLRLSCAASGRTFSNDALGWFRQAPRKEREFVAAIN
WNSGTYYADSVKGRFTISRDNAKNTVYLQMNSLKPEDTAVYSCAAASDYGLPRED
FLYDYWGQGTQVTVSSTS

SEQ ID NO. 26: Re9G12
GSQVQLVESGGGLVQAGGSLRLSCAASGRTFSSYVMGWFRQAPGKEREFVAHIS
WSGDSTYYADSVKGRFTIFRDNAKNTAYLQMNSLKPEDTAVYYCAADRGASYYYT
WASEYNYWGQGTQVTVSSTS

SEQ ID NO. 30: Re11H04
GSQVQLVESGGGLVQPGGSLRLSCAASGFTLDYYTIAWFRQAPGKEREGVSCISG
NDGSTYYADSVKGRFTISRDNAKNTVYLQMNSLKPEDTAVYYCAADRGESYYPFR
PSEYHYWGQGTQVTVSSTS

SEQ ID NO. 34: Re5D06
GSQVQLVESGGGLVQPGGSLRLSCAASGITLDYYAIGWFRQAPGKEREGVSRIRS
SDGSTNYADSVKGRFTMSRDNAKNTVYLQMNSLKPEDTAVYYCAYGPLTKYGSS
WYWPYEYDYWGQGTQVTVSSTS

SEQ ID NO. 38: Re6A11-spacer-RBD fusion
GSESEGSQVQLVESGGGLVQAGGSLRLSCAASGRTFSNDALGWFRQAPRKEREF
VAAINWNSGTYYADSVKGRFTISRDNAKNTVYLQMNSLKPEDTAVYSCAAASDYGL
PREDFLYDYWGQGTLVTVSSTSEGSEGPESSDGSDSTDPGEQGEGADASDGSEG
SSEGSEGPNITNLCPFGEVFNATRFASVYAWNRKRISNCVADYSVLYNSASFSTFK
CYGVSPTKLNDLCFTNVYADSFVIRGDEVRQIAPGQTGKIADYNYKLPDDFTGCVIA
WNSNNLDSKVGGNYNYLYRLFRKSNLKPFERDISTEIYQAGSTPCNGVEGFNCYFP
LQSYGFQPTNGVGYQPYRVVVLSFELLHAPATVCGP

SEQ ID NO. 39: His14-SUMO-Re6A11-spacer-RBD fusion
MSEEHHHHSGHHHTGHHHHSGSHHHGSEGSESSDSETNQEAEPESEPETEPETH
INLKVSDGSSEIFFKIKKTTPLRRLMEAFAKRQGKEMDSLRFLYDGIRIQADQTPEDL
DMEDNDIIEAQREQIGGGSESEGSQVQLVESGGGLVQAGGSLRLSCAASGRTFSN
DALGWFRQAPRKEREFVAAINWNSGTYYADSVKGRFTISRDNAKNTVYLQMNSLK
PEDTAVYSCAAASDYGLPREDFLYDYWGQGTLVTVSSTSEGSEGPESSDGSDSTD
PGEQGEGADASDGSEGSSEGSEGPNITNLCPFGEVFNATRFASVYAWNRKRISNC
VADYSVLYNSASFSTFKCYGVSPTKLNDLCFTNVYADSFVIRGDEVRQIAPGQTGKI
ADYNYKLPDDFTGCVIAWNSNNLDSKVGGNYNYLYRLFRKSNLKPFERDISTEIYQ
AGSTPCNGVEGFNCYFPLQSYGFQPTNGVGYQPYRVVVLSFELLHAPATVCGP

SEQ ID NO. 40: Spacer
EGSEGPESSDGSDSTDPGEQGEGADASDGSEGSSEGSEG

SEQ ID NO. 41: DsbG
MLKKILLLALLPAIAFAEELPAPVKAIEKQGITIIKTFDAPGGMKGYLGKYQDMGVTIYL
TPDGKHAISGYMYNEKGENLSNTLIEKEIYAPAGREMWQRMEQSHWLLDGKKDAP
VIVYVFADPFCPYCKQFWQQARPWVDSGKVQLRTLLVGVIKPESPATAAAILASKD
PAKTWQQYEASGGKLKLNVPANVSTEQMKVLSDNEKLMDDLGANVTPAIYYMSKE
NTLQQAVGLPDQKTLNIIMGNK

### References

Bessette, P. H., Åslund, F., Beckwith, J., and Georgiou, G. (1999). Efficient folding of proteins with multiple disulfide bonds in the Escherichia coli cytoplasm. Proceedings of the National Academy of Sciences 96, 13703-13708.
Chen, J., Miao, L., Li, J. M., Li, Y. Y., Zhu, Q. Y., Zhou, C. L., Fang, H. Q., and Chen, H. P. (2005). Receptor-binding domain of SARS-Cov spike protein: soluble expression in E. coli, purification and functional characterization. World J Gastroenterol 11, 6159-6164.
Frey, S., and Görlich, D. (2014a). A new set of highly efficient, tag-cleaving proteases for purifying recombinant proteins. J Chromatogr A 1337, 95-105.
Frey, S., and Görlich, D. (2014b). Purification of protein complexes of defined subunit stoichiometry using a set of orthogonal, tag-cleaving proteases. J Chromatogr A 1337, 106-115.
Jegouic, S. M., Loureiro, S., Thom, M., Paliwal, D., and Jones, I. M. (2020). Recombinant SARS-CoV-2 spike proteins for sero-surveillance and epitope mapping. bioRxiv
Lan, J., Ge, J., Yu, J., Shan, S., Zhou, H., Fan, S., Zhang, Q., Shi, X., Wang, Q., Zhang, L., and Wang, X. (2020). Structure of the SARS-CoV-2 spike receptor-binding domain bound to the ACE2 receptor. Nature 581, 215-220.
Levy, R., Weiss, R., Chen, G., Iverson, B. L., and Georgiou, G. (2001). Production of correctly folded Fab antibody fragment in the cytoplasm of Escherichia coli trxB gor mutants via the coexpression of molecular chaperones. Protein expression and purification 23, 338-347.
Wang, Q., Zhang, Y., Wu, L., Niu, S., Song, C., Zhang, Z., Lu, G., Qiao, C., Hu, Y., Yuen, K. Y., Wang, Q., Zhou, H., Yan, J., and Qi, J. (2020). Structural and Functional Basis of SARS-CoV-2 Entry by Using Human ACE2. Cell 181, 894-904.e9.
Yan, R., Zhang, Y., Li, Y., Xia, L., Guo, Y., and Zhou, Q. (2020). Structural basis for the recognition of SARS-CoV-2 by full-length human ACE2. Science 367, 1444-1448.
Zhou, P., Yang, X. L., Wang, X. G., Hu, B., Zhang, L., Zhang, W., Si, H. R., Zhu, Y., Li, B., Huang, C. L., Chen, H. D., Chen, J., Luo, Y., Guo, H., Jiang, R. D., Liu, M. Q., Chen, Y., Shen, X. R., Wang, X., Zheng, X. S., Zhao, K., Chen, Q. J., Deng, F., Liu, L. L., Yan, B., Zhan, F. X., Wang, Y. Y., Xiao, G. F., and Shi, Z. L. (2020a). A pneumonia outbreak associated with a new coronavirus of probable bat origin. Nature 579, 270-273.
Zhou, T., Tsybovsky, Y., Gorman, J., Rapp, M., Cerutti, G., Chuang, G. Y., Katsamba, P. S., Sampson, J. M., Schön, A., Bimela, J., Boyington, J. C., Nazzari, A., Olia, A. S., Shi, W., Sastry, M., Stephens, T., Stuckey, J., Teng, I. T., Wang, P., Wang, S., Zhang, B., Friesner, R. A., Ho, D. D., Mascola, J. R., Shapiro, L., and Kwong, P. D. (2020b). Cryo-EM Structures of SARS-CoV-2 Spike without and with ACE2 Reveal a pH-Dependent Switch to Mediate Endosomal Positioning of Receptor-Binding Domains. Cell Host Microbe 28, 867-879.e5.

## Claims

1. A method for the recombinant production of a polypeptide of interest in a host cell, e.g. a prokaryotic host cell, or a eukaryotic host cell comprising the steps:
(a) providing a host cell comprising (i) a nucleic acid molecule encoding a fusion polypeptide comprising the polypeptide of interest and a fold promoter, or (ii) a first nucleic acid molecule encoding a polypeptide of interest and a second nucleic acid molecule encoding a fold promoter for the polypeptide of interest, wherein the fold promoter is a VHH antibody directed against the polypeptide of interest; and
(b) cultivating the host cell under conditions where (i) the fusion polypeptide comprising the polypeptide of interest and the fold promoter is expressed or (ii) the polypeptide of interest and the fold promoter are co-expressed, and
(c) obtaining the polypeptide of interest in correctly folded form from the host cell.

2. The method of claim 1, wherein the host cell is a prokaryotic host cell, e.g. a Gram-negative prokaryotic cell such as *E. coli* or a Gram-positive prokaryotic cell such as *Bacillus ssp.*

3. The method of claim 1 or 2, wherein polypeptide of interest is expressed as a fusion polypeptide.

4. The method of any one of claims 1-3, wherein the nucleic acid molecule (i) encodes a fusion polypeptide further comprising an N-terminal tag, particularly a cleavable tag, and/or a heterologous spacer between the polypeptide of interest and the fold promoter, wherein the spacer particularly has a negative net charge of at least -5, and more particularly a content of at least about 15%, at least about 20% or least about 25% of negatively charged amino acids selected from Asp and Glu.

5. The method of claim 1 or 2, wherein the polypeptide of interest and the fold promoter are expressed as separate polypeptides.

6. The method of any one of the preceding claims, wherein the polypeptide of interest is the RBD of a coronavirus spike protein, e.g. the RBD of the SARS-Cov-1 spike protein, the RBD of the MERS-CoV spike protein, or the RBD of the SARS-CoV-2 spike protein, and particularly the RBD of the SARS-CoV-2 spike protein, particularly having an amino acid sequence as shown in SEQ: ID NO. 1 or a sequence identity of at least 80%, at least 90% or at least 95% thereto.

7. The method of claim 6, wherein the fold promoter is selected from a VHH antibody comprising
(a) a CDR3 sequence as shown in SEQ ID NO. 5, 9, 13, 17, 21, 25, 29, or 33,
(b) a CDR3 sequence, which has an identity of at least 80%, at least 90% or at least 95% to a CDR3 sequence of (a), or
(c) a VHH antibody, which competes with a VHH antibody of (a) for the binding to the SARS-CoV-2 spike protein S1 domain, particularly the receptor-binding domain (RBD) of the SARS-CoV-2 S1 domain.

8. A nucleic molecule encoding a fusion polypeptide comprising a polypeptide of interest and a fold promoter for the polypeptide of interest, wherein the fold promoter is a VVH antibody directed against the polypeptide of interest.

9. A set of nucleic molecules comprising a first nucleic acid molecule encoding a polypeptide of interest and a second nucleic acid molecule encoding a fold promoter for the polypeptide of interest, wherein the fold promoter is a VVH antibody directed against the polypeptide of interest.

10. A host cell comprising a nucleic acid molecule of claim 8, or a set of nucleic acid molecules of claim 9.

11. An RBD of the SARS-CoV-2 spike protein, particularly having an amino acid sequence as shown in SEQ: ID NO. 1 or a sequence identity of at least 80%, at least 90% or at least 95% thereto, in correctly folded form, particularly produced in a prokaryotic host cell, which binds to the non-competing VHH antibody Re5D06 having an amino acid sequence as shown in SEQ: ID NO. 34.

12. A non-covalent complex comprising an RBD of a coronavirus spike protein, particularly the RBD of the SARS-CoV-2 spike protein, more particularly having an amino acid sequence as shown in SEQ: ID NO. 1 or a sequence identity of at least 80%, at least 90% or at least 95% thereto, and a fold-enhancing VHH antibody.

13. A covalent fusion protein comprising an RBD of a coronavirus spike protein, particularly the SARS-CoV-2 spike protein, more particularly having an amino acid sequence as shown in SEQ: ID NO. 1 or a sequence identity of at least 80%, at least 90% or at least 95% thereto, and a fold-enhancing VHH antibody

14. An RBD of any one of claims 11-13 for use in medicine, particularly for use as a vaccine, or for use in the production of antibodies, particularly neutralizing antibodies.

15. A polypeptide for use as a vaccine, which is attached to a heterologous amino acid sequence having a length of at least about 5 amino acids and up to about 100 amino acids, and which has a negative net charge of at least -5 and particularly a content of at least about 15%, at least about 20% or least about 25% of negatively charged amino acids selected from Asp and Glu.


SEQUENCE LISTING

<110>  Max-Planck-Gesellschaft zur Förderung der Wissenschaften
       e.V.

<120>  Fold promoters and their use for the production and stabilization
        of polypeptides

<130>  72346P EP

<160>  41

<170>  PatentIn version 3.5

<210>  1
<211>  198
<212>  PRT
<213>  artificial sequence

<220>
<223>  SARS-CoV-2 RBD

<400>  1

Pro Asn Ile Thr Asn Leu Cys Pro Phe Gly Glu Val Phe Asn Ala Thr
1               5                   10                  15

Arg Phe Ala Ser Val Tyr Ala Trp Asn Arg Lys Arg Ile Ser Asn Cys
            20                  25                  30

Val Ala Asp Tyr Ser Val Leu Tyr Asn Ser Ala Ser Phe Ser Thr Phe
        35                  40                  45

Lys Cys Tyr Gly Val Ser Pro Thr Lys Leu Asn Asp Leu Cys Phe Thr
    50                  55                  60

Asn Val Tyr Ala Asp Ser Phe Val Ile Arg Gly Asp Glu Val Arg Gln
65                  70                  75                  80

Ile Ala Pro Gly Gln Thr Gly Lys Ile Ala Asp Tyr Asn Tyr Lys Leu
                85                  90                  95

Pro Asp Asp Phe Thr Gly Cys Val Ile Ala Trp Asn Ser Asn Asn Leu
            100                 105                 110

Asp Ser Lys Val Gly Gly Asn Tyr Asn Tyr Leu Tyr Arg Leu Phe Arg
        115                 120                 125

Lys Ser Asn Leu Lys Pro Phe Glu Arg Asp Ile Ser Thr Glu Ile Tyr
    130                 135                 140

Gln Ala Gly Ser Thr Pro Cys Asn Gly Val Glu Gly Phe Asn Cys Tyr
145                 150                 155                 160

Phe Pro Leu Gln Ser Tyr Gly Phe Gln Pro Thr Asn Gly Val Gly Tyr
                165                 170                 175

Gln Pro Tyr Arg Val Val Val Leu Ser Phe Glu Leu Leu His Ala Pro
            180                 185                 190

Ala Thr Val Cys Gly Pro
        195

<210>  2
<211>  127
<212>  PRT
<213>  artificial sequence

<220>
<223>  Re6A11

<400>  2

Gly Ser Gln Val Gln Leu Val Glu Ser Gly Gly Gly Leu Val Gln Ala
1               5                   10                  15

Gly Gly Ser Leu Arg Leu Ser Cys Ala Ala Ser Gly Arg Thr Phe Ser
            20                  25                  30

Asn Asp Ala Leu Gly Trp Phe Arg Gln Ala Pro Arg Lys Glu Arg Glu
        35                  40                  45

Phe Val Ala Ala Ile Asn Trp Asn Ser Gly Thr Tyr Tyr Ala Asp Ser
    50                  55                  60

Val Lys Gly Arg Phe Thr Ile Ser Arg Asp Asn Ala Lys Asn Thr Val
65                  70                  75                  80

Tyr Leu Gln Met Asn Ser Leu Lys Pro Glu Asp Thr Ala Val Tyr Ser
                85                  90                  95

Cys Ala Ala Ala Ser Asp Tyr Gly Leu Pro Arg Glu Asp Phe Leu Tyr
            100                 105                 110

Asp Tyr Trp Gly Gln Gly Thr Leu Val Thr Val Ser Ser Thr Ser
        115                 120                 125

<210>  3
<211>  9
<212>  PRT
<213>  artificial sequence

<220>
<223>  Re6A11 CDR1

<400>  3

Arg Thr Phe Ser Asn Asp Ala Leu Gly
1               5


<210>  4
<211>  14
<212>  PRT
<213>  artificial sequence

<220>
<223>  Re6A11 CDR2

<400>  4

Phe Val Ala Ala Ile Asn Trp Asn Ser Gly Thr Tyr Tyr Ala
1               5                   10


<210>  5
<211>  18
<212>  PRT
<213>  artificial sequence

<220>
<223>  Re6A11 CDR3

<400>  5

Ala Ala Ala Ser Asp Tyr Gly Leu Pro Arg Glu Asp Phe Leu Tyr Asp
1               5                   10                  15


Tyr Trp



<210>  6
<211>  131
<212>  PRT
<213>  artificial sequence

<220>
<223>  Re6B07

<400>  6

Gly Ser Gln Val Gln Leu Val Glu Ser Gly Gly Gly Leu Val Gln Pro
1               5                   10                  15


Gly Gly Ser Leu Arg Leu Ser Cys Ala Ala Ser Gly Phe Thr Leu Asp
            20                  25                  30


Tyr Tyr Ala Ile Gly Trp Phe Arg Gln Ala Pro Gly Lys Glu Arg Glu
        35                  40                  45


Gly Val Ser Tyr Ile Arg Ser Ser Asp Gly Thr Thr Tyr Tyr Ala Asp
    50                  55                  60

Ser Val Lys Gly Arg Phe Thr Ile Ser Arg Asp Asn Ala Lys Asn Thr
65                  70                  75                  80

Val Tyr Leu Gln Met Asn Ser Leu Lys Pro Glu Asp Thr Ala Val Tyr
                85                  90                  95

Tyr Cys Ala Ala Asp Glu Ala Tyr Tyr Ser Glu Leu Gly Trp Glu Ser
            100                 105                 110

Pro Trp Gly Trp Ser Tyr Trp Gly Gln Gly Thr Arg Val Thr Val Ser
        115                 120                 125

Ser Thr Ser
    130

<210>  7
<211>  9
<212>  PRT
<213>  artificial sequence

<220>
<223>  Re6B07 CDR1

<400>  7

Phe Thr Leu Asp Tyr Tyr Ala Ile Gly
1               5

<210>  8
<211>  15
<212>  PRT
<213>  artificial sequence

<220>
<223>  Re6B07 CDR2

<400>  8

Gly Val Ser Tyr Ile Arg Ser Ser Asp Gly Thr Thr Tyr Tyr Ala
1               5                   10                  15

<210>  9
<211>  21
<212>  PRT
<213>  artificial sequence

<220>
<223>  Re6B07 CDR3

<400>  9

Ala Ala Asp Glu Ala Tyr Tyr Ser Glu Leu Gly Trp Glu Ser Pro Trp
1               5                   10                  15

Gly Trp Ser Tyr Trp
                20

<210>  10
<211>  131
<212>  PRT
<213>  artificial sequence

<220>
<223>  Re7E02

<400>  10

Gly Ser Gln Val Gln Leu Val Glu Ser Gly Gly Gly Leu Val Gln Ala
1               5                   10                  15

Gly Gly Ser Leu Arg Leu Ser Cys Ala Ala Ser Gly Phe Thr Leu Asp
            20                  25                  30

Tyr Tyr Ala Ile Gly Trp Phe Arg Gln Ala Pro Gly Lys Glu Arg Glu
        35                  40                  45

Gly Val Ser Tyr Ile Arg Ser Ser Asp Gly Thr Thr Tyr Tyr Ala Asp
    50                  55                  60

Ser Val Lys Gly Arg Phe Thr Ile Ser Arg Asp Asn Ala Lys Asn Thr
65                  70                  75                  80

Val Tyr Leu Gln Met Asn Ser Leu Lys Pro Glu Asp Thr Ala Val Tyr
                85                  90                  95

Tyr Cys Ala Ala Asp Glu Ala Tyr Tyr Ser Glu Leu Gly Trp Glu Ser
            100                 105                 110

Pro Trp Gly Trp Ser Tyr Trp Gly Gln Gly Thr Gln Val Thr Val Ser
        115                 120                 125

Ser Thr Ser
    130

<210>  11
<211>  9
<212>  PRT
<213>  artificial sequence

<220>
<223>  Re7E02 CDR1

<400>  11

Phe Thr Leu Asp Tyr Tyr Ala Ile Gly
1               5

<210>  12
<211>  15
<212>  PRT
<213>  artificial sequence

<220>
<223>  Re7E02 CDR2

<400>  12

Gly Val Ser Tyr Ile Arg Ser Ser Asp Gly Thr Thr Tyr Tyr Ala
1               5                   10                  15


<210>  13
<211>  21
<212>  PRT
<213>  artificial sequence

<220>
<223>  Re7E02 CDR3

<400>  13

Ala Ala Asp Glu Ala Tyr Tyr Ser Glu Leu Gly Trp Glu Ser Pro Trp
1               5                   10                  15


Gly Trp Ser Tyr Trp
            20


<210>  14
<211>  116
<212>  PRT
<213>  artificial sequence

<220>
<223>  Re7H02

<400>  14

Gly Ser Gln Val Gln Leu Val Glu Ser Gly Gly Gly Leu Val Gln Ala
1               5                   10                  15


Gly Gly Ser Leu Arg Leu Ser Cys Ala Ala Ser Gly Arg Ala Phe Glu
            20                  25                  30


Ser Ala Pro Met Ser Trp Phe Arg Gln Ala Pro Gly Lys Glu Arg Glu
        35                  40                  45


Phe Val Ala Ser Val Ser Trp Ser Gly Asp Ser Thr Asn Tyr Ala Asp
    50                  55                  60


Ser Val Lys Gly Arg Phe Thr Ile Ser Arg Asp Asn Ala Glu Asn Thr
65                  70                  75                  80

Gly Tyr Leu Gln Met Asn Ser Leu Lys Pro Glu Asp Thr Ala Val Tyr
                85                  90                  95

Tyr Cys Lys Arg Gly Pro Tyr Trp Gly Gln Gly Thr Gln Val Thr Val
            100                 105                 110

Ser Ser Thr Ser
        115

<210>  15
<211>  9
<212>  PRT
<213>  artificial sequence

<220>
<223>  Re7H02 CDR1

<400>  15

Arg Ala Phe Glu Ser Ala Pro Met Ser
1               5

<210>  16
<211>  15
<212>  PRT
<213>  artificial sequence

<220>
<223>  Re7H02 CDR2

<400>  16

Phe Val Ala Ser Val Ser Trp Ser Gly Asp Ser Thr Asn Tyr Ala
1               5                   10                  15

<210>  17
<211>  6
<212>  PRT
<213>  artificial sequence

<220>
<223>  Re7H02 CDR3

<400>  17

Lys Arg Gly Pro Tyr Trp
1               5

<210>  18
<211>  131
<212>  PRT
<213>  artificial sequence

<220>
<223>  Re9C07

<400>  18

Gly Ser Gln Val Gln Leu Val Glu Ser Gly Gly Gly Leu Val Gln Ala
1               5                   10                  15

Gly Gly Ser Leu Arg Leu Ser Cys Ala Ala Ser Gly Arg Thr Phe Ser
            20                  25                  30

Arg Tyr Ala Met Gly Trp Phe Arg Gln Ala Pro Gly Lys Glu Arg Glu
        35                  40                  45

Phe Val Ala Ala Ile Thr Trp Asn Ala Asp Thr Thr Tyr Tyr Ala Asp
    50                  55                  60

Ser Val Lys Gly Arg Phe Thr Ile Ser Arg Asp Asn Ala Lys Asn Thr
65                  70                  75                  80

Val Tyr Leu Gln Met Asn Ser Leu Lys Pro Glu Asp Thr Ala Val Tyr
                85                  90                  95

Tyr Cys Ala Ala Gly Gly Asn His Tyr Tyr Ser Arg Ser Tyr Tyr Ser
            100                 105                 110

Ser Leu Glu Tyr Asp His Trp Gly Gln Gly Thr Gln Val Thr Val Ser
        115                 120                 125

Ser Thr Ser
    130


<210>  19
<211>  9
<212>  PRT
<213>  artificial sequence

<220>
<223>  Re9C07 CDR1

<400>  19

Arg Thr Phe Ser Arg Tyr Ala Met Gly
1               5


<210>  20
<211>  15
<212>  PRT
<213>  artificial sequence

<220>
<223>  Re9C07 CDR2

<400>  20

Phe Val Ala Ala Ile Thr Trp Asn Ala Asp Thr Thr Tyr Tyr Ala
1               5                   10                  15

<210>  21
<211>  21
<212>  PRT
<213>  artificial sequence

<220>
<223>  Re9C07 CDR3

<400>  21

Ala Ala Gly Gly Asn His Tyr Tyr Ser Arg Ser Tyr Tyr Ser Ser Leu
1               5                   10                  15

Glu Tyr Asp His Trp
            20

<210>  22
<211>  129
<212>  PRT
<213>  artificial sequence

<220>
<223>  Re9D02

<400>  22

Gly Ser Gln Val Gln Leu Val Glu Ser Gly Gly Gly Leu Val Gln Ala
1               5                   10                  15

Gly Gly Ser Leu Arg Leu Ser Cys Ala Ala Ser Gly Arg Thr Phe Ser
            20                  25                  30

Asn Tyr Ala Met Gly Trp Phe Arg Gln Ala Pro Gly Lys Glu Arg Glu
        35                  40                  45

Phe Val Ala Ala Ile Ser Trp Gly Gly Asp Thr Thr Tyr Tyr Ala Asp
    50                  55                  60

Ser Leu Lys Gly Arg Phe Thr Ile Ser Arg Asp Asn Ala Lys Asn Thr
65                  70                  75                  80

Val Tyr Leu Gln Met Asn Ser Leu Lys Pro Glu Asp Thr Ala Val Tyr
                85                  90                  95

Tyr Cys Ala Ala Asp Arg Gly Leu Ser Tyr Tyr Tyr Asp Arg Val Thr
            100                 105                 110

Glu Tyr Asp Tyr Trp Gly Gln Gly Thr Gln Val Thr Val Ser Ser Thr
        115                 120                 125

Ser

<210>  23
<211>  9
<212>  PRT
<213>  artificial sequence

<220>
<223>  Re9D02 CDR1

<400>  23

Arg Thr Phe Ser Asn Tyr Ala Met Gly
1               5

<210>  24
<211>  15
<212>  PRT
<213>  artificial sequence

<220>
<223>  Re9D02 CDR2

<400>  24

Phe Val Ala Ala Ile Ser Trp Gly Gly Asp Thr Thr Tyr Tyr Ala
1               5                   10                  15

<210>  25
<211>  19
<212>  PRT
<213>  artificial sequence

<220>
<223>  Re9D02 CDR3

<400>  25

Ala Ala Asp Arg Gly Leu Ser Tyr Tyr Tyr Asp Arg Val Thr Glu Tyr
1               5                   10                  15

Asp Tyr Trp

<210>  26
<211>  129
<212>  PRT
<213>  artificial sequence

<220>
<223>  Re9G12

<400>  26

Gly Ser Gln Val Gln Leu Val Glu Ser Gly Gly Gly Leu Val Gln Ala
1               5                   10                  15

Gly Gly Ser Leu Arg Leu Ser Cys Ala Ala Ser Gly Arg Thr Phe Ser
                20                  25                  30

Ser Tyr Val Met Gly Trp Phe Arg Gln Ala Pro Gly Lys Glu Arg Glu
        35                  40                  45

Phe Val Ala His Ile Ser Trp Ser Gly Asp Ser Thr Tyr Tyr Ala Asp
    50                  55                  60

Ser Val Lys Gly Arg Phe Thr Ile Phe Arg Asp Asn Ala Lys Asn Thr
65                  70                  75                  80

Ala Tyr Leu Gln Met Asn Ser Leu Lys Pro Glu Asp Thr Ala Val Tyr
                85                  90                  95

Tyr Cys Ala Ala Asp Arg Gly Ala Ser Tyr Tyr Tyr Thr Trp Ala Ser
            100                 105                 110

Glu Tyr Asn Tyr Trp Gly Gln Gly Thr Gln Val Thr Val Ser Ser Thr
        115                 120                 125

Ser

<210>  27
<211>  9
<212>  PRT
<213>  artificial sequence

<220>
<223>  Re9G12 CDR1

<400>  27

Arg Thr Phe Ser Ser Tyr Val Met Gly
1               5

<210>  28
<211>  15
<212>  PRT
<213>  artificial sequence

<220>
<223>  Re9G12 CDR2

<400>  28

Phe Val Ala His Ile Ser Trp Ser Gly Asp Ser Thr Tyr Tyr Ala
1               5                   10                  15

<210>  29
<211>  19
<212>  PRT

<213>  artificial sequence

<220>
<223>  Re9G12 CDR3

<400>  29

Ala Ala Asp Arg Gly Ala Ser Tyr Tyr Tyr Thr Trp Ala Ser Glu Tyr
1               5                   10                  15

Asn Tyr Trp

<210>  30
<211>  129
<212>  PRT
<213>  artificial sequence

<220>
<223>  Re11H04

<400>  30

Gly Ser Gln Val Gln Leu Val Glu Ser Gly Gly Gly Leu Val Gln Pro
1               5                   10                  15

Gly Gly Ser Leu Arg Leu Ser Cys Ala Ala Ser Gly Phe Thr Leu Asp
            20                  25                  30

Tyr Tyr Thr Ile Ala Trp Phe Arg Gln Ala Pro Gly Lys Glu Arg Glu
        35                  40                  45

Gly Val Ser Cys Ile Ser Gly Asn Asp Gly Ser Thr Tyr Tyr Ala Asp
    50                  55                  60

Ser Val Lys Gly Arg Phe Thr Ile Ser Arg Asp Asn Ala Lys Asn Thr
65                  70                  75                  80

Val Tyr Leu Gln Met Asn Ser Leu Lys Pro Glu Asp Thr Ala Val Tyr
                85                  90                  95

Tyr Cys Ala Ala Asp Arg Gly Glu Ser Tyr Tyr Pro Phe Arg Pro Ser
            100                 105                 110

Glu Tyr His Tyr Trp Gly Gln Gly Thr Gln Val Thr Val Ser Ser Thr
        115                 120                 125

Ser

<210>  31
<211>  9

<212>  PRT
<213>  artificial sequence

<220>
<223>  Re11H04 CDR1

<400>  31

Phe Thr Leu Asp Tyr Tyr Thr Ile Ala
1               5


<210>  32
<211>  15
<212>  PRT
<213>  artificial sequence

<220>
<223>  Re11H04 CDR2

<400>  32

Gly Val Ser Cys Ile Ser Gly Asn Asp Gly Ser Thr Tyr Tyr Ala
1               5                   10                  15


<210>  33
<211>  19
<212>  PRT
<213>  artificial sequence

<220>
<223>  Re11H04 CDR3

<400>  33

Ala Ala Asp Arg Gly Glu Ser Tyr Tyr Pro Phe Arg Pro Ser Glu Tyr
1               5                   10                  15


His Tyr Trp




<210>  34
<211>  131
<212>  PRT
<213>  artificial sequence

<220>
<223>  Re5D06

<400>  34

Gly Ser Gln Val Gln Leu Val Glu Ser Gly Gly Gly Leu Val Gln Pro
1               5                   10                  15


Gly Gly Ser Leu Arg Leu Ser Cys Ala Ala Ser Gly Ile Thr Leu Asp
            20                  25                  30


Tyr Tyr Ala Ile Gly Trp Phe Arg Gln Ala Pro Gly Lys Glu Arg Glu

35                  40                  45

Gly Val Ser Arg Ile Arg Ser Ser Asp Gly Ser Thr Asn Tyr Ala Asp
    50                  55                  60

Ser Val Lys Gly Arg Phe Thr Met Ser Arg Asp Asn Ala Lys Asn Thr
65                  70                  75                  80

Val Tyr Leu Gln Met Asn Ser Leu Lys Pro Glu Asp Thr Ala Val Tyr
                85                  90                  95

Tyr Cys Ala Tyr Gly Pro Leu Thr Lys Tyr Gly Ser Ser Trp Tyr Trp
            100                 105                 110

Pro Tyr Glu Tyr Asp Tyr Trp Gly Gln Gly Thr Gln Val Thr Val Ser
        115                 120                 125

Ser Thr Ser
    130

<210>  35
<211>  9
<212>  PRT
<213>  artificial sequence

<220>
<223>  Re5D06 CDR1

<400>  35

Ile Thr Leu Asp Tyr Tyr Ala Ile Gly
1               5

<210>  36
<211>  15
<212>  PRT
<213>  artificial sequence

<220>
<223>  Re5D06 CDR2

<400>  36

Gly Val Ser Arg Ile Arg Ser Ser Asp Gly Ser Thr Asn Tyr Ala
1               5                   10                  15

<210>  37
<211>  21
<212>  PRT
<213>  artificial sequence

<220>
<223>  Re5D06 CDR3

<400>  37

Ala Tyr Gly Pro Leu Thr Lys Tyr Gly Ser Ser Trp Tyr Trp Pro Tyr
1               5                   10                  15

Glu Tyr Asp Tyr Trp
            20

<210>  38
<211>  369
<212>  PRT
<213>  artificial sequence

<220>
<223>  Re6A11-spacer-RBD fusion

<400>  38

Gly Ser Glu Ser Glu Gly Ser Gln Val Gln Leu Val Glu Ser Gly Gly
1               5                   10                  15

Gly Leu Val Gln Ala Gly Gly Ser Leu Arg Leu Ser Cys Ala Ala Ser
            20                  25                  30

Gly Arg Thr Phe Ser Asn Asp Ala Leu Gly Trp Phe Arg Gln Ala Pro
        35                  40                  45

Arg Lys Glu Arg Glu Phe Val Ala Ala Ile Asn Trp Asn Ser Gly Thr
    50                  55                  60

Tyr Tyr Ala Asp Ser Val Lys Gly Arg Phe Thr Ile Ser Arg Asp Asn
65                  70                  75                  80

Ala Lys Asn Thr Val Tyr Leu Gln Met Asn Ser Leu Lys Pro Glu Asp
                85                  90                  95

Thr Ala Val Tyr Ser Cys Ala Ala Ala Ser Asp Tyr Gly Leu Pro Arg
            100                 105                 110

Glu Asp Phe Leu Tyr Asp Tyr Trp Gly Gln Gly Thr Leu Val Thr Val
        115                 120                 125

Ser Ser Thr Ser Glu Gly Ser Glu Gly Pro Glu Ser Ser Asp Gly Ser
    130                 135                 140

Asp Ser Thr Asp Pro Gly Glu Gln Gly Glu Gly Ala Asp Ala Ser Asp
145                 150                 155                 160

Gly Ser Glu Gly Ser Ser Glu Gly Ser Glu Gly Pro Asn Ile Thr Asn
                165                 170                 175

Leu Cys Pro Phe Gly Glu Val Phe Asn Ala Thr Arg Phe Ala Ser Val
            180                 185                 190

Tyr Ala Trp Asn Arg Lys Arg Ile Ser Asn Cys Val Ala Asp Tyr Ser
        195                 200                 205

Val Leu Tyr Asn Ser Ala Ser Phe Ser Thr Phe Lys Cys Tyr Gly Val
    210                 215                 220

Ser Pro Thr Lys Leu Asn Asp Leu Cys Phe Thr Asn Val Tyr Ala Asp
225                 230                 235                 240

Ser Phe Val Ile Arg Gly Asp Glu Val Arg Gln Ile Ala Pro Gly Gln
                245                 250                 255

Thr Gly Lys Ile Ala Asp Tyr Asn Tyr Lys Leu Pro Asp Asp Phe Thr
            260                 265                 270

Gly Cys Val Ile Ala Trp Asn Ser Asn Asn Leu Asp Ser Lys Val Gly
        275                 280                 285

Gly Asn Tyr Asn Tyr Leu Tyr Arg Leu Phe Arg Lys Ser Asn Leu Lys
    290                 295                 300

Pro Phe Glu Arg Asp Ile Ser Thr Glu Ile Tyr Gln Ala Gly Ser Thr
305                 310                 315                 320

Pro Cys Asn Gly Val Glu Gly Phe Asn Cys Tyr Phe Pro Leu Gln Ser
                325                 330                 335

Tyr Gly Phe Gln Pro Thr Asn Gly Val Gly Tyr Gln Pro Tyr Arg Val
            340                 345                 350

Val Val Leu Ser Phe Glu Leu Leu His Ala Pro Ala Thr Val Cys Gly
        355                 360                 365

Pro

<210>  39
<211>  498
<212>  PRT
<213>  artificial sequence

<220>
<223>  His14-SUMO-Re6A11-spacer-RBD fusion

<400>  39

Met Ser Glu Glu His His His His Ser Gly His His His Thr Gly His
1               5                   10                  15

His His His Ser Gly Ser His His His Gly Ser Glu Gly Ser Glu Ser
            20                  25                  30

Ser Asp Ser Glu Thr Asn Gln Glu Ala Glu Pro Glu Ser Glu Pro Glu
        35                  40                  45

Thr Glu Pro Glu Thr His Ile Asn Leu Lys Val Ser Asp Gly Ser Ser
    50                  55                  60

Glu Ile Phe Phe Lys Ile Lys Lys Thr Thr Pro Leu Arg Arg Leu Met
65                  70                  75                  80

Glu Ala Phe Ala Lys Arg Gln Gly Lys Glu Met Asp Ser Leu Arg Phe
                85                  90                  95

Leu Tyr Asp Gly Ile Arg Ile Gln Ala Asp Gln Thr Pro Glu Asp Leu
            100                 105                 110

Asp Met Glu Asp Asn Asp Ile Ile Glu Ala Gln Arg Glu Gln Ile Gly
        115                 120                 125

Gly Gly Ser Glu Ser Glu Gly Ser Gln Val Gln Leu Val Glu Ser Gly
    130                 135                 140

Gly Gly Leu Val Gln Ala Gly Gly Ser Leu Arg Leu Ser Cys Ala Ala
145                 150                 155                 160

Ser Gly Arg Thr Phe Ser Asn Asp Ala Leu Gly Trp Phe Arg Gln Ala
                165                 170                 175

Pro Arg Lys Glu Arg Glu Phe Val Ala Ala Ile Asn Trp Asn Ser Gly
            180                 185                 190

Thr Tyr Tyr Ala Asp Ser Val Lys Gly Arg Phe Thr Ile Ser Arg Asp
        195                 200                 205

Asn Ala Lys Asn Thr Val Tyr Leu Gln Met Asn Ser Leu Lys Pro Glu
    210                 215                 220

Asp Thr Ala Val Tyr Ser Cys Ala Ala Ala Ser Asp Tyr Gly Leu Pro
225                 230                 235                 240

Arg Glu Asp Phe Leu Tyr Asp Tyr Trp Gly Gln Gly Thr Leu Val Thr
                245                 250                 255

Val Ser Ser Thr Ser Glu Gly Ser Glu Gly Pro Glu Ser Ser Asp Gly
            260                 265                 270

Ser Asp Ser Thr Asp Pro Gly Glu Gln Gly Glu Gly Ala Asp Ala Ser
        275                 280                 285

Asp Gly Ser Glu Gly Ser Ser Glu Gly Ser Glu Gly Pro Asn Ile Thr
    290                 295                 300

Asn Leu Cys Pro Phe Gly Glu Val Phe Asn Ala Thr Arg Phe Ala Ser
305                 310                 315                 320

Val Tyr Ala Trp Asn Arg Lys Arg Ile Ser Asn Cys Val Ala Asp Tyr
                325                 330                 335

Ser Val Leu Tyr Asn Ser Ala Ser Phe Ser Thr Phe Lys Cys Tyr Gly
            340                 345                 350

Val Ser Pro Thr Lys Leu Asn Asp Leu Cys Phe Thr Asn Val Tyr Ala
        355                 360                 365

Asp Ser Phe Val Ile Arg Gly Asp Glu Val Arg Gln Ile Ala Pro Gly
    370                 375                 380

Gln Thr Gly Lys Ile Ala Asp Tyr Asn Tyr Lys Leu Pro Asp Asp Phe
385                 390                 395                 400

Thr Gly Cys Val Ile Ala Trp Asn Ser Asn Asn Leu Asp Ser Lys Val
                405                 410                 415

Gly Gly Asn Tyr Asn Tyr Leu Tyr Arg Leu Phe Arg Lys Ser Asn Leu
            420                 425                 430

Lys Pro Phe Glu Arg Asp Ile Ser Thr Glu Ile Tyr Gln Ala Gly Ser
        435                 440                 445

Thr Pro Cys Asn Gly Val Glu Gly Phe Asn Cys Tyr Phe Pro Leu Gln
    450                 455                 460

Ser Tyr Gly Phe Gln Pro Thr Asn Gly Val Gly Tyr Gln Pro Tyr Arg
465                 470                 475                 480

Val Val Val Leu Ser Phe Glu Leu Leu His Ala Pro Ala Thr Val Cys
                485                 490                 495

Gly Pro

<210>  40
<211>  39
<212>  PRT
<213>  artificial sequence

<220>
<223>  Spacer

<400>  40

Glu Gly Ser Glu Gly Pro Glu Ser Ser Asp Gly Ser Asp Ser Thr Asp
1               5                   10                  15

Pro Gly Glu Gln Gly Glu Gly Ala Asp Ala Ser Asp Gly Ser Glu Gly
            20                  25                  30

Ser Ser Glu Gly Ser Glu Gly
        35

<210>  41
<211>  0
<212>  PRT
<213>  artificial sequence

<220>
<223>  DsbG protein

<400>  41
000